# EUROPEAN PATENT APPLICATION

(11) **EP 2 161 580 A1**
(43) Date of publication of application: **10.03.2010**
(21) Application number: 08163482.6
(22) Date of filing: 02.09.2008
(51) Int. Cl.: G01N 33/92

(54) **Biomarkers for identifying subjects predisposed to developing insulin resistance and determining the insulin resistance status**

(71) Applicant: Nederlandse Organisatie voor toegepast-natuurwetenschappelijk onderzoek TNO, 2628 VK Delft (NL)
(72) Inventor: Wopereis, Suzan, 2628 VK Delft (NL)
(74) Representative: Hatzmann, Martin

(57) **Abstract**

The invention is related to method for determining whether a healthy individual has an increased risk of developing insulin resistance. In one aspect, the invention comprises the determination of a relative level of at least two fatty acids in a sample of the individual. In a further aspect, the invention comprises the determination of a level of activity of stearoyl-CoA desaturase (SCD) in a sample of said individual. The invention further provides a method of determining a degree of insulin resistance in an individual.

## Description

The invention relates to methods for determining a predisposition of an individual for developing diabetes mellitus type 2, especially insulin resistance. More specifically, the invention provides methods and means for determining a risk for developing diabetes mellitus type 2, especially insulin resistance, of a healthy individual. Furthermore, the invention provides methods and means for determining the status of insulin resistance in an individual. The invention also relates to kits that can be used for determining a predisposition for developing insulin resistance in a healthy individual and for determining the insulin resistance status in an individual.

Diabetes mellitus type 2 or Type 2 Diabetes (DM2) is a metabolic disorder that is primarily characterized by insulin resistance, relative insulin deficiency, and hyperglycemia. It is rapidly increasing in the developed world, and there is some evidence that this pattern will be followed in much of the rest of the world in coming years. The CDC (Centers for Disease Control and Prevention) has characterized the increase as an epidemic. In addition, whereas this disease used to be seen primarily in adults over age 45, it is now increasingly seen in children and adolescents, an increase thought to be linked to rising rates of obesity in these age groups.

DM2 is a chronic, progressive disease that has no clearly established cure. It may go unnoticed for years in a patient before diagnosis, since the initial symptoms are typically mild and can be sporadic. However, severe complications can result from improperly managed DM2, including retinopathy, nephropathy, and wounds that are slow to heal, and cardiovascular disease.

DM2 is presently of largely unknown etiology. It is generally thought that insulin resistance (IR) is a major factor in the pathogenesis of DM2. IR is the condition in which normal amounts of insulin are inadequate to produce a normal insulin response from fat, muscle and liver. IR in fat tissue results in hydrolysis of stored triglycerides, which elevates free fatty acids in the blood plasma. IR in muscle reduces glucose uptake, whereas IR in liver reduces glucose storage, with both effects resulting in elevated blood glucose.

High plasma levels of insulin and glucose due to IR often lead to metabolic syndrome (also termed insulin resistance syndrome or Syndrome X) and DM2. IR is frequently found in people with visceral adiposity (i.e., a high degree of fatty tissue underneath the abdominal muscle wall) as distinct from subcutaneous adiposity.

Signs and features of IR are hypertension, fasting hyperglycemia and impaired glucose tolerance, dyslipidemia involving elevated triglycerides, small dense low-density lipoprotein particles and decreased HDL cholesterol levels, and elevated uric acid levels. More recently, chronically elevated levels of systemic inflammation markers such as C-reactive protein (CRP), fibrinogen, interleukin 6 (IL-6), and tumor necrosis factor-alpha (TNFa) have been found to be associated with the risk of future IR disease. Some studies have pointed to oxidative stress due to a variety of causes including dietary fructose mediated increased uric acid levels.

Currently, it is only possible to diagnose and treat people that already have IR and/or diabetes type 2. It would be a major benefit if the onset of disease could be prevented. Therefore, there is a clear need for the development of a test that identifies a subject at risk of or predisposed to developing IR. The biomarker profile described in this application is likely to become an important aid in the prevention of the development of IR, as it identifies individuals with IR susceptibility.

The invention provides a method for determining whether a healthy individual has an increased risk of developing insulin resistance, the method comprising determining in a sample of said individual a relative level of at least two of said fatty acids, and determining from said relative level whether the individual has an increased risk.

Insulin resistance is a state in which a given concentration of insulin produces a less-than-expected biological effect. The pathogenesis of insulin resistance is still unclear. The syndromes of insulin resistance actually comprise a broad clinical spectrum, which includes obesity, glucose intolerance, diabetes, and syndrome X, as well as an extreme insulin-resistant state. Many of these disorders are associated with various endocrine, metabolic, and genetic conditions. These syndromes may also be associated with immunological diseases and may exhibit distinct phenotypic characteristics.

Whether or not a human individual suffers from IR is generally determined by diagnostic criteria. Direct and indirect methods of varying complexity are currently employed for these purposes. Non-limiting examples are hyperinsulinemic euglycemic glucose clamp (direct), insulin suppression test (direct), intravenous glucose tolerance test (indirect), quantitative insulin sensitivity index (QUICKI; indirect), homeostatic model assessment (HOMA; indirect), 1/insulin, and Matusda index, among others (Muniyappa R et al. Am J Physiol Endocrinol Metab, 2008; 294(1): E15-26). Often insulin resistance is accompanied by a blood pressure of more than 130/85 mm Hg, a low level of high-density-lipoprotein (HDL) cholesterol (less than 40 mg/dL in men and less than 50 mg/dL in women), and/or high levels of triglycerides of more than 150 mg/dL. Further indicators that are often used include abdominal obesity, the presence of acanthosis nigricans, and the personal and family medical history of the individual.

A method of the invention provides a test whether a healthy individual has a predisposition to develop IR. A healthy individual is defined as an individual not suffering from, and having no indication of suffering from, IR. A healthy individual, therefore, will have normal results in a screening test that is used to assess whether the individual is suffering from IR.

The relative level of at least two fatty acids is preferably determined in a sample comprising a body fluid selected from blood, serum, blood plasma, including umbilical cord blood, serum and blood plasma, and urine, or in a tissue sample such as adipose tissue and placenta, whereby a most preferred sample comprises blood plasma. The levels of saturated and monounsaturated fatty acids can be readily determined in these samples.

Preferred fatty acids comprise free fatty acids. Preferred free fatty acids comprise palmitoleic acid (C16:1) and palmitate (C16:0). It was found by the inventors that a reduced ratio of palmitoleic acid (C16:1) to palmitate (C16:0) in a sample is indicative for a healthy individual being at risk of, or predisposed to, developing IR.

It is preferred to compare the relative level of at least two fatty acids determined in the healthy individual with a relative level of said fatty acids in a corresponding reference. With a corresponding reference is meant a sample that is taken from the same tissue. It is further preferred that the corresponding reference comprises an average relative level of said fatty acids in a population. The relevant data from one or more references can be stored in a database such as a database that is present in an electronic storage device. Preferably, said database comprises relevant data concerning the relative level of said fatty acids in one or more individuals, of which the IR status has been determined. A threshold can be used to discriminate between a relative level of said fatty acids in a sample of an individual that is likely to develop IR and a relative level of said fatty acids in a sample of an individual that is not likely to develop IR. It will be evident to a skilled person that more than one threshold can be used to discriminate between individuals with a high or low risk of developing IR, and an intermediate group of individuals. The relative level of said fatty acids in the reference can be determined prior to, after or in parallel to the determination of a relative level of said fatty acids in the sample of the healthy individual.

Further preferred free fatty acids comprise oleate (C18:1) and stearate (C18:0). Also for these fatty acids, it was found by the investigators that a reduced ration of oleate (C18:1) to stearate (C18:0) in a sample is indicative for a healthy individual being at risk of, or predisposed to, developing IR.

Of these fatty acids, the ratio between (C16:1) and (C16:0) is a more sensitive index for a predisposition for developing IR because of the lower dietary proportion of (C16:1) compared with (C18:1), which means that the ratio between (C16:1) and (C16:0) is less influenced by food intake.

A preferred method of the invention comprises determining the fatty acids palmitoleic acid (C16:1), palmitate (C16:0), oleate (C18:1) and stearate (C18:0).

The amounts of palmitoleic acid (C16:1), palmitate (C16:0), oleate (C18:1) and stearate (C18:0) in a sample such as blood, blood plasma, blood serum and urine can be determined by methods known in the art. For example, they can be determined by high performance liquid chromatography and gas chromatography using various detection systems (e.g. FID, MS) and derivatization methods (e.g. methylesthers), and thin layer chromatography.

It was further found that the inflammatory status of the individual is indicative of a risk for an individual of developing insulin resistance. A pro-inflammatory status of an individual was found to be indicative of an increased risk of developing IR, whereby the relative amounts of the free fatty acids omega 6 and omega 3 was used as a measure for the inflammatory status in a body fluid.

Therefore, the fatty acids in a further preferred method of the invention comprise omega-6 and omega-3 fatty acids. The relative amounts of these fatty acids in a tissue such as a body fluid selected from blood, including umbilical cord blood, serum, blood plasma, and urine, or in tissue samples such as adipose tissue and placenta, can be determined by methods known in the art, including high performance liquid chromatography and gas chromatography using various detection systems (e.g. FID, MS) and derivatization methods (e.g. methylesthers), and thin layer chromatography

It is preferred that the ratio of omega-6 to omega-3 fatty acids is determined by determining the relative amounts of (C18:2) and (C22:6) fatty acids.

In a preferred method of the invention, a pro-inflammatory status is combined with the relative amounts of palmitoleic acid (C16:1) and palmitate (C16:0), and/or of oleate (C18:1) and stearate (C18:0). The combination resulted in an improved correlation with a predisposition for IR.

Therefore, a further preferred method of the invention comprises determining the fatty acids palmitoleic acid (C16:1), palmitate (C16:0), oleate (C18:1) and stearate (C18:0), in combination with the determination of a ratio between omega-3 and omega-6 fatty acids, whereby said omega 3 and omega 6 fatty acids preferably are polyunsaturated fatty acids.

The invention further provides a kit that can be used for determining a predisposition for developing insulin resistance in a healthy individual. Said kit comprises means for determining a relative level of at least two fatty acids in a sample of the individual, whereby it is preferred that said fatty acids are free fatty acids. Said kit preferably comprises means for isolating a sample, preferably a blood sample, from said individual, means for storage of said sample to prevent lipolysis, and means for isolating fatty acids such as, for example, isopropanol. Said kit preferably also comprises one or more standards that can be used for the identification of fatty acids. Preferred standards are selected from C17:0 lyso-phosphatidylcholine, di-C12:0 phosphatidylcholine, tri-C17:0 glycerol ester, C17:0 cholesterol ester and heptadecanoic acid (C17:0).

In a further embodiment, a method of the invention comprises determining in a sample from the individual a level of activity of a stearoyl-CoA desaturase (SCD), and determining whether the individual has an increased risk for developing insulin resistance on the basis of the determined activity of SCD.

SCD is an integral membrane protein of the endoplasmic reticulum. SCD catalyzes the formation of monounsaturated fatty acid whereby a cis-double bond is inserted in the Δ9 position of the fatty acid substrate. The ratio of monounsaturated product/substrate is a measure for the activity of SCD.

There are three isoforms of SCD in human, SCD1, SCD2 and SCD5 (Zhang et al. 2005. Biochem J. 388: 135; Wang J et al. 2005 Biochem Biophys Res Commun. 332(3):735-42) while mice have four Scd isoforms, Scd1 through Scd4 (Zhang et al. (1999); Wang et al., 2005). SCD1 shares about 85% amino acid identity with all 4 mouse Scd isoforms, SCD2 shares 57% homology with the rodent Scd-2 (and 61% with human SCD-1), while SCD5 has no rodent orthologues. The expression of the mouse isoforms varies among different tissues. Scd1 is the main isoform expressed in mouse liver, Scd 2 is the main isoform expressed in brain and adipose tissue, Scd3 is expressed exclusively in skin, whereas Scd4 is expressed predominantly in the heart (Zhang et al. 2005. Biochem J. 388: 135). The level of activity of SCD1 and SCD2 in human and Scd1 and Scd2 in mice is most relevant for a method of the invention.

It is preferred that the sample from the individual for determining a level of activity of a stearoyl-CoA desaturase (SCD), preferably Scd1 and Scd2 in mouse and SCD1 and SCD2 in human, comprises liver, muscle, peripheral blood mononuclear cells (PBMCs), placenta, umbilical cord and/or adipose tissue such as white adipose tissue and brown adipose tissue from the healthy individual. A most preferred sample comprises white adipose tissue.

The activity of SCD can be determined by methods known in the art. For example, an in vitro assay for determining desaturase activity comprises the conversion of radiolabelled saturated fatty acyl-CoAs into the corresponding mono-unsaturated fatty acids, and resolution of the components by TLC (Zhang et al. 2005. Biochem J. 388: 135).

In one embodiment, a method of the invention uses the determination of the enzyme activity of SCD for determining a predisposition for IR in sample from a healthy individual. An indication of the activity of SCD can also be provided by measuring the steady state level of the enzyme, and/or by determining the presence of activators such as co-factors, or inhibitors in the sample.

It is preferred to compare the level of activity of SCD determined in the healthy individual with a level of activity determined in a corresponding reference. With a corresponding reference is meant a sample that is taken from the same tissue. The level of activity in the reference can be determined prior to, after or in parallel to the determination of the activity in the sample of the healthy individual.

It is further preferred that the reference comprises an average level of activity of a SCD in a population. The relevant data from one or more references can be stored in a database such as a database that is present in an electronic storage device. Preferably, said database comprises relevant data concerning the activity of SCD in one or more individuals, of which the IR status has been determined. A threshold can be used to discriminate between a level of activity of SCD in a sample of an individual that is likely to develop IR and a level of activity of SCD in a sample of an individual that is not likely to develop IR. It will be evident to a skilled person that more than one threshold can be used to discriminate between individuals with a high or low risk of developing IR, and an intermediate group of individuals.

SCD is an important metabolic control point, and increased levels of activity have often been associated with obesity and diabetes. Inhibition of its expression is thought to be beneficial in the treatment of obesity, diabetes, cardiovascular disease, and other metabolic diseases (Dobrzyn and Ntambi 2004 Trends Card Med 14: 77).

The present inventors, however, have found that a healthy individual who is not suffering from IR and has none of the criteria indicative of IR, with a reduced activity of SCD is predisposed to developing IR, while a healthy individual with a normal or increased activity of SCD has a normal or reduced risk for developing IR. A reference in a preferred method of the invention therefore comprises an average level of activity of a SCD in a population. A low level of activity of SCD in a sample of an individual, as compared to the level of activity of SCD in the reference, is indicative of a predisposition of said individual for developing IR.

In one embodiment of the invention, it is preferred that the healthy individual has taken a diet prior to the provision of a sample for determining the activity of SCD, the diet resulting in reduced food intake (reduced ingestion). Said diet preferably is a low-fat and fish free diet. The diet can be associated with increased physical activity such as walking or riding a bike for 30 minutes each day. The diet is preferably followed for a period between one hour and 1 month, more preferred between 12 hours and 1 week, most preferred for about 24 hours before the provision of a sample.

The diet preferably comprises, by itself or in addition, the absence of food intake for a period of time prior to the determination of a relative level of at least two fatty acids in a sample of said individual. Said period of time is between 1 hour and 48 hours, more preferred between 8 hours and 12 hours, most preferred about 10 hours.

The method of the invention is used for determining a predisposition for developing IR in a mammal. Said mammal preferably comprises a livestock animal, a pet animal, and/or a human, including but not limited to horse, sheep, goat, mouse, pig, dog, cat, camel, sheep, goat, monkey, chimpanzee, hamster, rat, rabbit, cow, guinea pig and human. A preferred mammal is a human.

Said mammal can be of any age, including a baby, a child, an adolescent, and an adult. A preferred mammal is a baby.

The invention further provided the use of a relative level of at least two fatty acids in a sample of an individual for determining whether the individual has an increased risk for developing insulin resistance, whereby it is preferred that the fatty acids comprise palmitoleic acid (C16:1) and palmitate (C16:0), and/or of oleate (C18:1) and stearate (C18:0). Further preferred fatty acids comprise omega-3 and omga-6 fatty acids, such as (C18:2) and (C22:6) fatty acids.

The invention further provides a method of determining the status of insulin resistance in an individual, the method comprising determining in a sample from the individual a level of expression of at least one gene selected from Table 1, and determining whether the individual has an increased degree of insulin resistance on the basis of the determined level of expression of the gene.

Currently, the diagnostic methods used for determining the degree of IR, or IR status, of individuals are invasive and complex. It would be a major benefit if the IR status of an individual could be determined in a single blood sample. Therefore, there is a clear need for the development of a simple non-invasive test that determines the degree of insulin resistance of an individual.

The present inventors further surprisingly found that the expression level of at least one gene selected from Table 1 correlates with the degree of IR. This correlation was already evident in healthy individuals, and persevered during the development of IR. As indicated in Table 1, the correlation was either positive or negative, whereby a positive correlation means that an elevated expression is indicative for an individual developing or having a higher degree of IR, and whereby a negative correlation means that a reduced expression is indicative for an individual developing or having a higher degree of IR.

The expression level of a gene selected from Table 1 can be determined in samples comprising nucleated cells, such as blood cells, or in a tissue sample such as liver, placenta, umbilical cord, muscle and adipose tissue, whereby a most preferred sample comprises peripheral blood mononuclear cells (PBMCs).

An expression level of at least one gene selected from Acss2, Arfgap1, Tfrc, Cox6b2, Barhl2, Abcb4, Cyp4b1, Sars2, Fgf16, and Tcea18 is preferably determined at the mRNA or protein level, most preferred at the mRNA level. A protein expression level can be determined by a known method including Western blotting and immunohistochemistry. Methods for determining mRNA expression levels are known in the art and comprise Northern blotting, quantitative reverse transcriptase polymerase chain reaction (q-rt-PCR) and other linear or exponential amplification methods, and microarray analysis. In a preferred embodiment, a method of the invention comprises comparing a determined expression level of a gene selected from Table 1 to the expression level of the gene in a reference sample.

A preferred gene selected from Table 1 is Acss2. The present inventors surprisingly found that the expression level of Acss2 is negatively correlated to the degree of IR. A low expression level of Acss2 in a sample of an individual is indicative of a high degree of IR. The expression level of Accs2 is thus a biomarker for the insulin resistance status in an individual.

Acss2 is also known as ACAS, Acs1, Acas1, Acas2, AceCS1 and 1110017C11Rik and was identified as a protein acyl-CoA synthetase short-chain family member 2 (or acetyl-CoA synthetase 1, acetyl-Coenzyme A synthetase 1 (AMP forming), acetyl-Coenzyme A synthetase 2 (ADP forming), acetyl-Coenzyme A synthetase 2 (AMP forming)).

The genes acyl-CoA synthetase and stearoyl-CoA desaturase-1 (SCD-1) are known to be co-regulated by insulin and the inflammatory cytokine tumor necrosis factor α (TNF α) during and after adipocyte differentiation (Weiner FR et al. J Biol Chem. 1991; 266(35):23525-23528). Furthermore, both enzymes play key roles in the metabolism of fatty acids. The control of acyl-coA synthetase and SCD-1 expression might be the mechanism by which adipocytes can adjust their capacity for fatty acid metabolism in response to external stimuli.

Furthermore, acetyl CoA synthetase 2 is a mitochondrial matrix enzyme that provides acetyl-CoA for the production of energy that is utilized under ketogenic conditions, such as starvation and diabetes (Fujino T et al. J Biol Chem. 2001; 276(14):11420-11426). Under ketogenic conditions, relatively large amounts of fatty acid-derived free acetate are released from the liver in blood, which is also observed under conditions of starvation and in patients with diabetes.

Interestingly, a good correlation was found between Acss2 expression and concentrations of intermediates in the citrate cycle, such as succinate (66%); malate (68%); citrate (72%), isocitrate (75%) and fumarate (75%). See also Figure 10. Therefore, the Acss2 concentration can be used as a measure to determine the concentrations of intermediates from the citrate cycle in a tissue such as liver.

In a further embodiment, the invention provides a kit for determining a status of insulin resistance in an individual, the kit comprising means for determining a level of expression of at least one gene selected from Table 1 in a sample from the individual. In a preferred embodiment, said kit comprises one or more primers, an enzyme and a buffer for quantitative reverse transcriptase polymerase chain reaction (q-rt-PCR).

The invention further provides the use of a relative level of at least two fatty acids in a sample of an individual for determining whether the individual has an increased risk for developing insulin resistance, whereby it is preferred that the fatty acids comprise palmitoleic acid (C16:1) and palmitate (C16:0), and/or of oleate (C18:1) and stearate (C18:0). Further preferred fatty acids comprise omega-3 and omga-6 fatty acids, such as (C18:2) and (C22:6) fatty acids.

Figure legends
Figure 1. Results from glucose tolerance test at day 0 (start) and week 12. Shown is the average of the area under the glucose curve per animal.
Figure 2. (A) Results of a PLS model in which LC-MS FFA data from day 0 are correlated to the glucose area under the curve (AUC) on week 12. (B) Score plot of the PLS model that identifies the most important free fatty acids in the model. The height of the bar corresponds with the importance of the fatty acid with the glucose AUC.
Figure 3. Mode of action of stearyl-SoA desaturase (SCD).
Figure 4. Scatter plots of SCD activity on week 0 and glucose AUC on week 12. SCD activity was determined by determining the (C16:1) to (C16:0) ratio, and the (C18:1) to (C18:0) ratio, respectively.
Figure 5. Scatter plot of the (C18:2) to (C22:6) ratio on week 0 and glucose AUC on week 12.

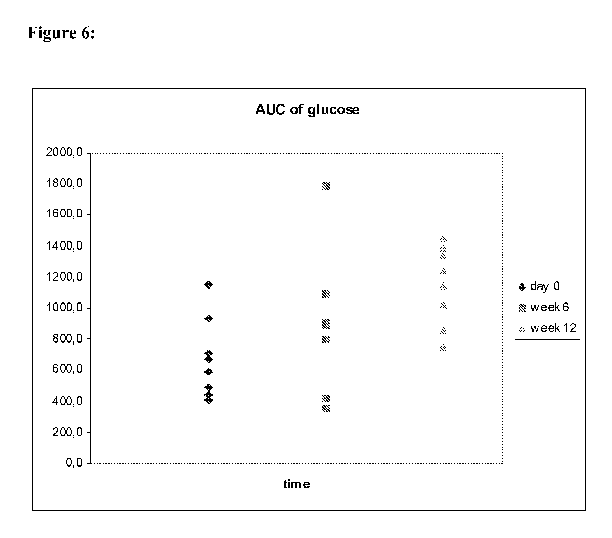

Figure 6. Results from glucose tolerance test of individual apoE3Leiden mice at day 0, week 6 and week 12. The average of the area under the glucose curve is shown.
Figure 7. Scatter plot of Acss2 expression on weeks 0, 6, 12 and glucose AUC on weeks 0, 6, and 12. A negative correlation was found between Acss2 expression and glucose AUC. The correlation was -0.79.

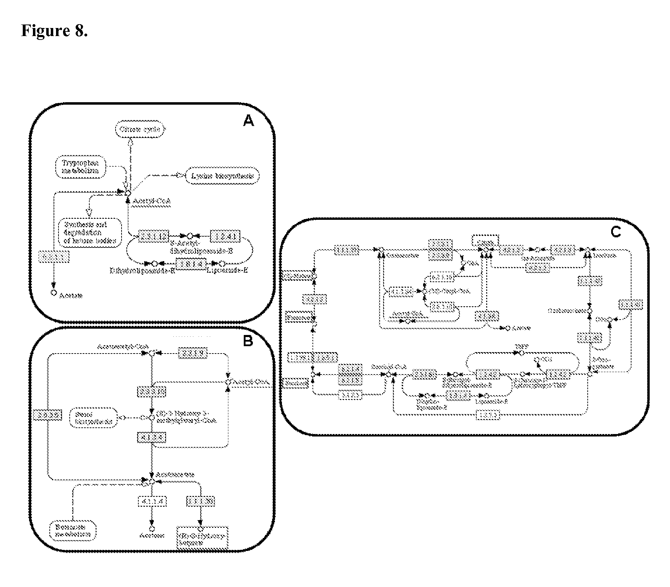

Figure 8. Acss2 related pathways. A) Acss2 (enzyme 6.2.1.1) converts acetate into acetyl-CoA and vice versa. Acetyl-CoA is involved in several pathways, such as synthesis and degradation of ketone bodies (shown in B) and the citrate cycle (shown in C). B) 3-hydroxybutyrate (in red box) was found to be significantly increased in the time-course analysed (week 6 versus week 0; week 12 versus week 6 and week 12 versus week 0). C) The metabolites citrate, succinate, fumarate and malate (in blue boxes) were found to be significantly increased in the time-course (week 6 versus week 0; week 12 versus week 6 and week 12 versus week 0)
Figure 9. Scatter plot of Acss2 expression on weeks 0, 6, 12 and liver metabolite concentrations on weeks 0, 6, 12. Positive correlations were found between Acss2 expression and liver metabolites deriving from citrate cycle. The correlations were respectively 66% for succinate, 68% for malate, 72% for citrate and 75% for fumarate.

**Table 1. Overview of biomarkers for the insulin resistance status. Biomarkers are based on gene expression data from PBMCs.**

| **EntrezID** | **Common name** | **Description** | **Correlation factor** |
|---|---|---|---|
| 60525 | Acss2 | acyl-CoA synthetase short-chain family member 2 | -0,79 |
| 228998 | Arfgap1 | ADP-ribosylation factor GTPase activating protein 1 | -0,76 |
| 22042 | Tfrc | transferrin receptor | 0,81 |
| 104382 | Cox6b2 | cytochrome c oxidase subunit VIb polypeptide 2 | 0,80 |
| 243187 | Barhl2 | - | 0,78 |
| 18670 | Abcb4 | ATP-binding cassette, sub-family B (MDR/TAP), member 4 | 0,78 |
| 13120 | Cyp4b1 | cytochrome P450, family 4, subfamily b, polypeptide 1 | 0,77 |
| 71984 | Sars2 | seryl-aminoacyl-tRNA synthetase 2 | 0,76 |
| 80903 | Fgf16 | fibroblast growth factor 16 | 0,76 |
| 66684 | Tceal8 | transcription elongation factor A (SII)-like 8 | 0,75 |

### Examples

### Example 1

To identify early plasma markers that are predictive for peripheral IR, male APOE*3Leiden (E3L) mice (n=15) were fed a high fat diet containing 24% beef tallow (HF diet; Hope Farms, Woerden, The Netherlands) for 12 weeks. Male ApoE*3-Leiden transgenic (E3L) mice were from TNO-Pharma, Gaubius Laboratory, Leiden, The Netherlands. The animals were housed in wire-topped Macrolon cages with a layer of sawdust as bedding in the light- and temperature-controlled animal facility of TNO. The mice had free access to water and diet and prior to the diet intervention they received standard laboratory chow (Hope Farms, Woerden, The Netherlands). At the start of the experimental treatment period, E3L mice were 12 weeks of age and fed. Blood was collected prior to the study (t=0) by tail bleeding into chilled paraoxon (Sigma, St. Louis, USA)-coated capillary tubes to prevent ongoing in vitro lipolysis. Tubes were placed on ice and centrifuged, and the plasma was collected for analysis with an LC-MS method for quantitative analysis of free fatty acids (FFA). Free fatty acids (FFA) were analyzed with electrospray LC-MS (Bijlsma S et al. 2006, Analytical Chemistry 78: 567-574; Verhoeckx KCM et al. 2004, International Immunopharmacology 4: 1499-1514) by extracting 10 µl of plasma with 300 µl of isopropanol containing several internal standards (IS: C17:0 lyso-phosphatidylcholine, di-C12:0 phosphatidylcholine, tri-C17:0 glycerol ester, C17:0 cholesterol ester and heptadecanoic acid (C17:0)). The sample was injected in a Thermo LTQ instrument equipped with a Thermo Surveyor HPLC pump. Detection of FFA is performed in negative ion mode. Data was acquired by scanning the instrument form m/z 300 to 1200 at a scan rate of approximately 2 scans/s.
At t=0 and t=12 weeks glucose tolerance test (GTT) were performed to measure the degree of IR. After a 4-h fast, mice were injected intraperitoneally with glucose (2 g/kg body weight) and blood glucose levels were monitored for 120 minutes using a hand-held glucose analyzer (FreeStyle, Disectronics, Vianen, The Netherlands). Partial least squares (PLS) was used to correlate the plasma free fatty acids from day 0 to the AUC of glucose on week 12 (Barker M et al., 2003, Journal of Chemometrics 17: 166-173). Single and double cross-validation was used to validate the PLS-DA models.

### Results

### Glucose tolerance test

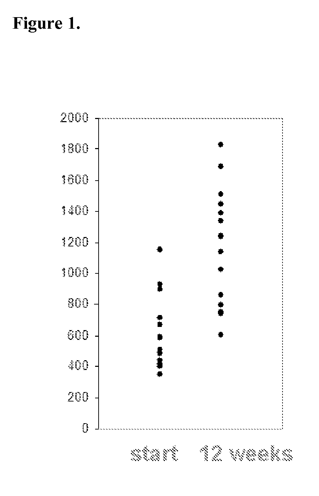

Figure 1 shows the results of the glucose tolerance tests. Per individual animal, the average area under the glucose curve (AUC) is shown prior to and after 12 weeks of high fat diet intervention. Although the glucose AUC showed that inbred apoE3Leiden mice on average developed peripheral IR after 12 weeks of diabetogenic diet intervention, still a large variation in individual glucose response is observed (Figure 1). Thus, despite the equal genetic background of the apoE3Leiden mice, phenotypical variation is observed resulting in differences in GTT response.

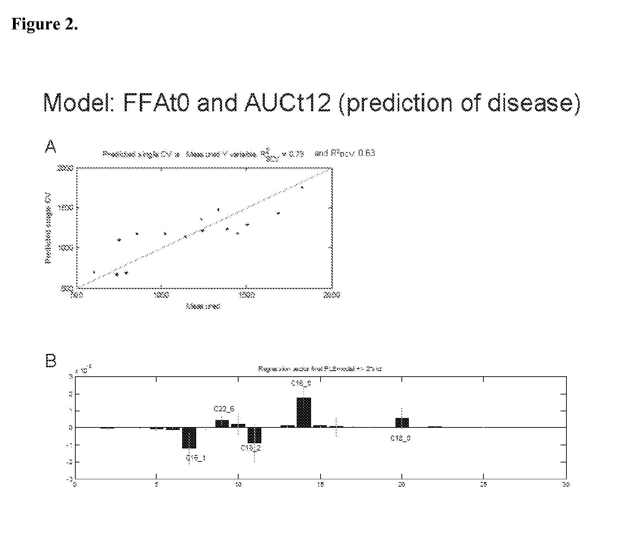

Multivariate data analysis: Partial Least Squares (PLS) was used to correlate the plasma free fatty acids from day 0 to the AUC of glucose on week 12. Single cross-validation and double cross validation was used to validate the PLS models. An association of 73% was found between the FFA data from day 0 and the glucose AUC on week 12 after single cross validation, and of 63% after double cross validation (Figure 2A). The fatty acids C16:0, C16:1, C18:2, C18:0 and C22:6 (out of a total of 28 fatty acids) in this order of magnitude were identified as most important for the correlation with the glucose AUC on week 12 (Figure 2B). C16:0 represents palmitic acid, C16:1 palmitoleic acid (n-7), C18:2 linoleic acid (n-6) and/or octadecanoic acid (n-9), C18:0 stearic acid and C22:6 docosahexaenoic acid (n-3).
It is likely that C18:2 represents linoleic acid (n-6), because octadecanoic acid is an intermediate product of elongation and desaturation reactions taking place in endoplasmic reticulum of liver and is therefore not secreted in plasma. Furthermore, linoleic acid (n-6) is the most abundant fatty acid species in chow diet (52%, data not shown).

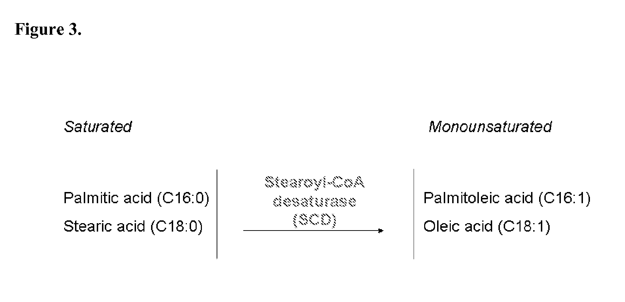

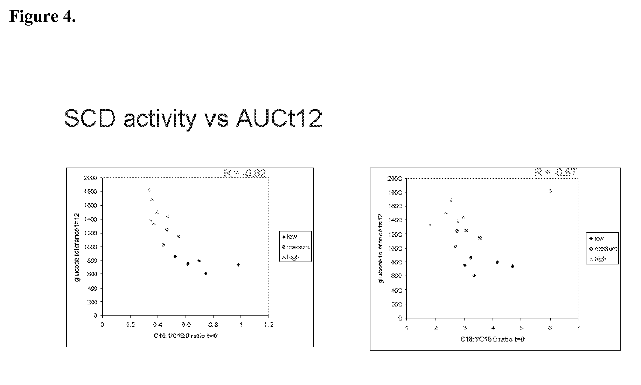

Plasma non-esterified fatty acids (NEFA) C16:1/C16:0 ratio is standard used as a measure for the steroyl-CoA desaturase (SCD) activity. Alternatively, NEFA C18:1/C18:0 ratio can be used. C16:1/C16:0 is thought to be a more sensitive index to detect any change in SCD activity because of the lower dietary proportion of C16:1 compared to C18:1. SCD is required for the desaturation of C16:0 and C18:0 to C16:1 and C18:1 respectively (Figure 3). For each mouse the activity of SCD was calculated on the basis of C16:1/C16:0 and the C18:1/C18:0 ratios and correlated to its glucose AUC on week 12, which resulted in negative correlations of -0.82 and -0.67 respectively (Figure 4).

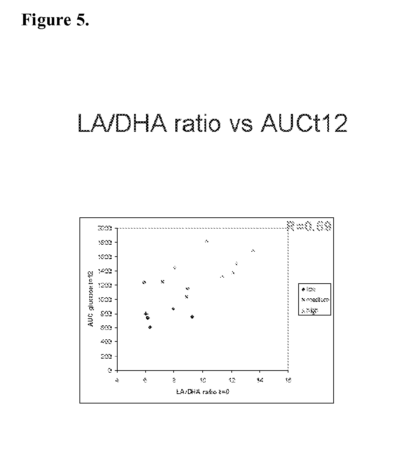

Omega-3 fatty acids are known as anti-inflammatory compounds, whereas omega-6 fatty acids are known as pro-inflammatory compounds. The plasma ratio C18:2 (n-6)/C22:6 (n-3) could therefore give information on the 'inflammatory status' of apoE3Leiden mice. This ratio was calculated for each individual mouse and correlated to its glucose AUC on week 12. This resulted in a positive correlation of 0.69 (Figure 5). From this result the hypothesis was drawn that apoE3Leiden mice with an offset phenotype characterized by a pro-inflammatory state in terms of the plasma proportion n-6 to n-3 develop a higher degree of whole body IR after 12 weeks of high fat diet intervention.

From these results it was concluded that apoE3Leiden mice with an offset phenotype characterized by a lower activity of SCD have a higher risk of whole body IR after 12 weeks of high fat diet intervention.

### Example 2

Male ApoE*3-Leiden transgenic (E3L) mice were from TNO-Pharma, Gaubius Laboratory, Leiden, The Netherlands. The animals were housed in wire-topped Macrolon cages with a layer of sawdust as bedding in the light- and temperature-controlled animal facility of TNO. The mice had free access to water and diet and prior to the diet intervention they received standard laboratory chow (Hope Farms, Woerden, The Netherlands). Male APOE*3Leiden (E3L) mice were fed a high fat diet containing 24% beef tallow (HF diet; Hope Farms, Woerden, The Netherlands). At the start of the experimental treatment period, E3L mice were 12 weeks of age and fed. Animals were euthanized with CO/CO2 after t=0, 6 weeks and 12 weeks (n=8 per time point) of high fat diet feeding to collect blood for transcriptome and livers for metabolome analysis. Erythrocytes were lysed with lysis solution and livers were snap-frozen immediately in liquid nitrogen and stored at -80°C until use. All experiments were approved by the Institutional Animal Care and Use Committee of TNO and were in compliance with European Community specifications regarding the use of laboratory animals

Total RNA from peripheral blood mononuclear cells (PBMCs) was extracted using RNAzol (Campro Scientific, Veenendaal, The Netherlands) and glass beads according to the manufacturer's instructions. The integrity of each RNA sample obtained was examined by Agilent Lab-on-a-chip technology using the RNA 6000 Nano LabChip kit and a bioanalyzer 2100 (Agilent Technologies, Amstelveen, The Netherlands). The One-Cycle Target Labelling and Control Reagent kit (Affymetrix #900493) and the protocols optimized by Affymetrix were used to prepare biotinylated cRNA (from 5µg of total RNA) for microarray hybridization (n = 8 per time point). The quality of intermediate products was again controlled using the RNA 6000 Nano Lab-on-a-chip and bioanalyzer 2100. Fragmented cRNA was mixed with spiked controls, applied to Affymetrix Test chips, and good quality samples were then used to hybridize with murine GeneChip® 430 2.0 arrays. The hybridization, probe array washing and staining, and washing procedures were executed as described in the Affymetrix protocols, and probe arrays were scanned with a Hewlett-Packard Gene Array Scanner (Leiden Genome Technology Center, Leiden, The Netherlands. Raw signal intensities (from CEL files) were normalized using the GCRMA algorithm (gc-rma slow). Expression data were log-transformed (base 2). Quality control of microarray data was performed using BioConductor packages (a.o. simpleaffy and affyplm, through the NuGO MadMax pipeline: https://madmax.bioinformatics.nl). For each tissue, raw signal intensities (from CEL files) were normalized using the GCRMA algorithm (gc-rma slow). Probesets were remapped and annotated into Entrez gene-ids using the custom MBNI CDF-file, version 9.0.1. (Dai et al., 2005). Genes were filtered on expression value above 5 in at least 4 samples. Expression data were log-transformed (base 2). Expression values of individual samples at week 1, 6, 9 and 12 were corrected for mean expression in samples of week 0, resulting in 2log ratios of expression compared to mean expression at week 0.

At t=0, t=6 and t=12 weeks glucose tolerance test (GTT) were performed to measure the degree of IR. After a 4-h fast mice were injected intraperitoneally with glucose (2 g/kg body weight) and blood glucose levels were monitored for 120 minutes using a hand-held glucose analyzer (FreeStyle, Disectronics, Vianen, The Netherlands).

### Liver metabolomics analysis:

The GC-MS method used for analyzing a broad range of metabolites was identical to the method reported for microbial metabolomics (Koek MM et al. (2006) Analytical Chemistry 78:1272-1281), except for the sample type. Lyophilized liver samples (5 mg tissue) were extracted with methanol and after evaporation the metabolites were derivatized (oximation and silylation).

### Results

### Glucose tolerance test

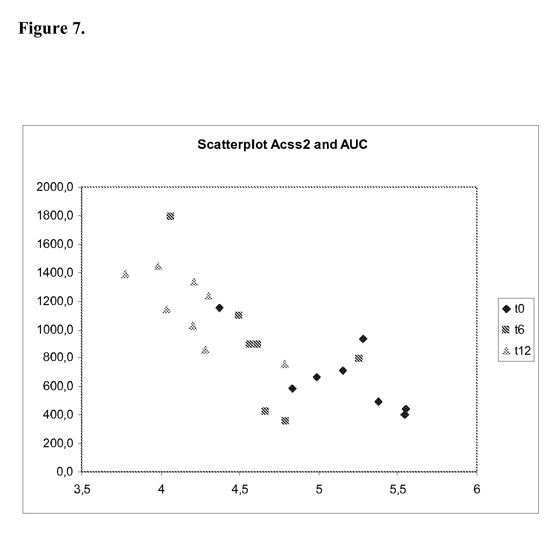

Figure 7 shows the results of the glucose tolerance tests. Per individual animal, the average area under the glucose curve (AUC) is shown at t=0, t=6 and t=12 weeks of high fat diet intervention. Although the glucose AUC showed that inbred apoE3Leiden mice on average developed peripheral IR after 12 weeks of diabetogenic diet intervention, still a large variation in individual glucose response is observed (Figure 7).

Thus, despite the equal genetic background of the apoE3Leiden mice, phenotypical variation is observed resulting in differences in GTT response.

### Transcriptomics:

One microarray from week 6 did not pass quality control criteria. Therefore, the microarray dataset consisted of 23 samples from PBMCs. After normalization, annotation and filtering of genes with a low expression value, 9599 genes could be used for correlation analysis.

Pearson correlation was used to correlate the PBMC transcriptome from day 0, week 6 and week 12 to the AUC of glucose on day 0, week 6 and week 12. Genes with a high correlation to the AUC of glucose are listed in Table 1. The gene Acss2 was identified as having the highest correlation coefficient. A negative association of 79% was found between the expression of Acss2 from day 0, weeks 6 and 12 and the glucose AUC on day 0, week 6 and 12 (Figure 8). The expression of Acss2 at the separate time points also resulted in negative correlations with the glucose AUC at the separate time points of respectively 76%, 68% and 78%.

These results indicate that individuals with a relatively lower expression level of Acss2 have a higher degree of insulin resistance. Acss2 expression is a good representative of the insulin resistance status in individuals. On average Acss2 expression was found to be significantly decreased in the time course.

### Liver metabolomics:

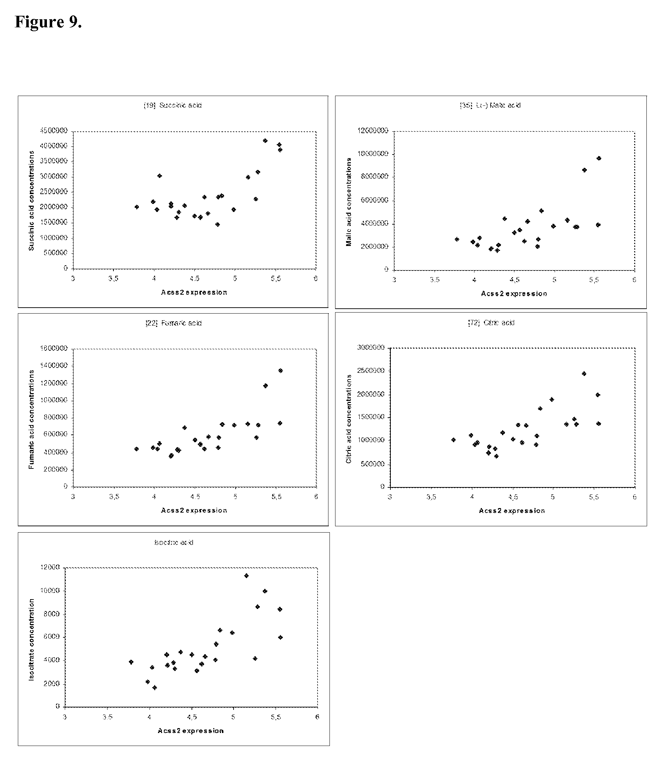

The hypothesis is that expression of PBMC Acss2 is related to the activity of the protein Acyl-coA synthetase in general. Liver metabolomics was used to test this hypothesis. Figure 9 shows the acetyl-coA synthetase related pathways and metabolites that were measured with the metabolomics platform in these pathways. Pearson correlation was used to correlate the Acss2 expression from PBMCs to concentrations of the metabolites malate, succinate, fumarate, citrate and 3-hydroxybutyrate. Interestingly, good correlations were found between Acss2 expression and concentrations of the metabolites measured from the citrate cycle (succinate 66%; malate 68%; citrate 72% and fumarate 75%; see also Figure 10).

This suggests a) that Acss2 expression is related to the activity of Acetyl-CoA synthetase and b) that expression of Acss2 in PBMCs is similar to expression of Acss2 in liver tissue. On average, concentrations of metabolites involved in citrate cycle were significantly decreased in the time course, suggesting that the development of insulin resistance is related to a decreased (liver) energy metabolism.

## Claims

1. A method for determining whether a healthy individual has an increased risk of developing insulin resistance, the method comprising:
determining in a sample of said individual a relative level of at least two fatty acids, and determining from said relative level whether the individual has in increased risk.

2. The method according to claim 1, whereby the sample comprises a body fluid selected from blood, serum, blood plasma.

3. The method according to claim 1 or claim 2, whereby the fatty acids comprise palmitoleic acid (C16:1) and palmitate (C16:0).

4. The method according to claim 1 or claim 2, whereby the fatty acids comprise oleate (C18:1) and stearate (C18:0).

5. The method of claim 1 or claim 2, whereby the fatty acids comprise palmitoleic acid (C16:1), palmitate (C16:0), oleate (C18:1) and stearate (C18:0).

6. The method of claim 1 or claim 2, whereby the fatty acids comprise omega-6 and omega-3 fatty acids.

7. The method of claim 6, whereby the omega-6 and omega 3 fatty acids comprise (C18:2) and (C22:6) fatty acids.

8. The method according to claim 1 or claim 2, whereby the fatty acids comprise palmitoleic acid (C16:1), palmitate (C16:0), oleate (C18:1), stearate (C18:0), (C18:2) and (C22:2) fatty acids.

9. A kit for determining a predisposition for developing insulin resistance in a healthy individual, said kit comprising means for determining a relative level of at least two fatty acids in a sample of the individual.

10. A method of determining whether an individual has an increased risk for developing insulin resistance, the method comprising:
determining in a sample from the individual a level of activity of a stearoyl-CoA desaturase (SCD);
determining whether the individual has an increased risk for developing insulin resistance on the basis of the determined activity of SCD.

11. The method of claim 9, whereby said sample comprises adipose tissue.

12. A method of determining a degree of insulin resistance in an individual, the method comprising:
determining in a sample from the individual a level of expression of at least one gene selected from Table 1;
determining whether the individual has an increased degree of insulin resistance on the basis of the determined level of expression of the at least one gene selected from Table 1.

13. The method of claim 12, wherein the sample comprises peripheral blood mononuclear cells.

14. The method of claim 12 or claim 13, wherein the at least one gene selected from Table 1 comprises Acss2.

15. The method according to any of the previous claims, whereby the individual has taken a diet prior to the provision of a sample.

16. The method of claim 15, whereby the diet comprises the absence of food intake.

17. The method of claim 15 or 16, whereby the diet is taken for a period of at least 12 hours prior to the provision of a sample.

18. The method according to any of the previous claims, whereby the individual is a livestock animal, a pet animal, or a human.

19. A kit for determining a status of insulin resistance in an individual, comprising means for determining a level of expression of at least one gene selected from Table 1 in a sample from the individual.

20. Use of a relative level of at least two fatty acids in a sample of an individual for determining whether the individual has an increased risk for developing insulin resistance.
